# EUROPÄISCHE PATENTANMELDUNG

(11) **EP 1 990 001 A1**
(43) Veröffentlichungstag der Anmeldung: **12.11.2008**
(21) Anmeldenummer: 07009367.9
(22) Anmeldetag: 10.05.2007
(51) Int. Cl.: A61B 5/15

(54) **Stechsystem und Lanzettenträgerband**

(71) Anmelder: Roche Diagnostics GmbH, 68305 Mannheim (DE); F.HOFFMANN-LA ROCHE AG, 4070 Basel (CH)
(72) Erfinder: Konya, Ahmet, 67165 Waldsee (DE); Deck, Frank, 67150 Niederkirchen (DE); List, Hans, 64754 Hesseneck-Keilbach (DE); Keil, Michael, 67071 Ludwigshafen (DE)
(74) Vertreter: Mommer, Niels

(57) **Zusammenfassung**

Die Erfindung betrifft ein Stechsystem mit einem Trägerband (2), das mehrere Lanzetten (3) trägt, einem Gerätegehäuse (9), das eine Fördereinrichtung (6), um die Lanzetten (3) durch Bewegen des Trägerbandes (2) in einer Förderrichtung nacheinander in eine Gebrauchsposition zu bringen, und einen Stechantrieb (7), um eine in der Gebrauchsposition positionierte Lanzette (3) für eine Stechbewegung zu beschleunigen, enthält und eine Gehäuseöffnung (8) zum Anlegen eines Körperteils, in das mit einer Stechbewegung einer Lanzette (3) eingestochen werden soll, aufweist, und eine Betätigungseinrichtung (11) zum Antreiben der Fördereinrichtung (6). Erfindungsgemäß hat das Stechsystem (1) eine lösbare Sperre (17, 18, 21), die einen Weitertransport des Trägerbandes (2) blockiert, sobald eine Lanzette (3) die Gebrauchsposition erreicht hat. Die Erfindung betrifft ferner ein Lanzettenträgerband (2) für ein derartiges Stechsystem.

## Beschreibung

Die Erfindung geht aus von einem Stechsystem mit den im Oberbegriff des Anspruchs 1 angegebenen Merkmalen.

Zu einem derartigen Stechsystem gehört als Lanzettenvorrat ein Trägerband, das mehrere Lanzetten trägt. Diese Lanzetten werden durch Bewegen des Trägerbandes in einer Förderrichtung nacheinander in eine Gebrauchsposition gebracht, in der sie mit einem Stechantrieb für eine Stechbewegung beschleunigt werden können, um in einem an eine Gehäuseöffnung des Stechsystems angelegten Körperteil eine Stichwunde zur Gewinnung einer Körperflüssigkeitsprobe zu erzeugen.

Derartige Stechsysteme werden beispielsweise von Diabetikern verwendet, die mehrmals täglich ihren Blutzuckerspiegel überprüfen müssen und dafür eine Körperflüssigkeitsprobe, in der Regel Blut oder interstitielle Flüssigkeit benötigen, die aus einer mit einem Stechsystem erzeugten Stichwunde gewonnen wird.

Im Gegensatz zu Stechsystem mit Trommelmagazinen, die typischerweise nur sechs oder acht Lanzetten enthalten, kann ein Trägerband einen Lanzettenvorrat mit einer wesentlich größeren Anzahl von Lanzetten bilden. Stechsysteme mit einem Lanzettenvorrat in Form eines Trägerbands haben deshalb den Vorteil, dass sich ein Benutzer seltener um den Austausch eines Trägerbandes oder, bei Verwendung von Wegwerfgeräten, um ein neues Gerät bemühen muss.

Damit mit einer Lanzette eines Trägerbandes in ein an eine Gehäuseöffnung angelegtes Körperteil eingestochen werden kann, muss die Lanzette in einer Gebrauchsposition in Bezug auf die Gehäuseöffnung positioniert werden.

Aufgabe der Erfindung ist es, einen Weg aufzuzeigen, wie bei einem Stechsystem der eingangs genannten Art Lanzetten präzise in einer Gebrauchsposition positioniert werden können.

Diese Aufgabe wird durch ein Stechsystem mit den im Anspruch 1 angegebenen Merkmalen sowie durch ein Trägerband mit den im Anspruch 12 angegebenen Merkmalen gelöst. Vorteilhafte Weiterbildungen der Erfindung sind Gegenstand der Unteransprüche.

Ein erfindungsgemäßes Stechsystem hat eine lösbare Sperre, die einen Weitertransport des Trägerbandes blockiert, sobald eine Lanzette die Gebrauchsposition erreicht hat. Das Stechsystem kann ein Stechgerät und austauschbare Trägerbänder mit Lanzetten umfassen. Das Stechsystem kann jedoch auch als ein Stechgerät ausgebildet sein, bei dem ein Austausch eines Trägerbandes nicht vorgesehen ist und das bestimmungsgemäß entsorgt wird, sobald alle Lanzette des Trägerbands benutzt wurden. Ein erfindungsgemäßes Trägerband hat Blockadeelemente, die zum Positionieren einer Lanzette in einer Gebrauchsposition mit einer lösbaren Sperre eines Stechgeräts zusammenwirken. Diese Blockadeelemente können beispielsweise Löcher sein, in welche die Sperre eingreift, um einen Weitertransport des Trägerbandes zu verhindern. Die Blockadeelemente können beispielsweise auch als quer zur Längsrichtung des Trägerbandes verlaufende Stufen ausgebildet sein, die an einen Anschlag des Stechgeräts anschlagen, sobald eine Lanzette eine Gebrauchsposition erreicht hat.

Bei einem erfindungsgemäßen Stechsystem wird eine Fördereinrichtung, mit der die Lanzetten durch Bewegen des Trägerbandes nacheinander in eine Gebrauchsposition gebracht werden können, bevorzugt über eine Rutschkupplung angetrieben. Sobald eine Lanzette ihre Gebrauchsposition erreicht hat, wird ein Weitertransport des Bandes durch eine Sperre blockiert. Die Rutschkupplung rutscht daraufhin durch.

Dies hat den Vorteil, dass eine Antriebsbewegung, die mit einer Betätigungseinrichtung zum Antreiben der Fördereinrichtung erzeugt wird, nicht auf den zum Positionieren einer Lanzette in der Gebrauchsposition erforderlichen Förderweg abgestimmt sein muss und deshalb auch größer sein kann. Der Antrieb der Fördereinrichtung eines erfindungsgemäßen Stechsystems kann deshalb vorteilhaft einfach aufgebaut sein und beispielsweise auch für andere Funktionen wie das Spannen einer Feder eines Stechantriebs, mit dem eine in der Gebrauchsposition positionierte Lanzette für eine Stechbewegung zu beschleunigt wird, genutzt werden.

Rutschkupplungen werden auch als Sicherheits- oder Überlastkupplungen bezeichnet. Im Rahmen der Anmeldung sind diese Begriffe gleichbedeutend. Derartige Kupplungen öffnen sich, sobald ein angreifendes Drehmoment einen vorgegebenen Grenzwert überschreitet und übertragen folglich nur Drehmomente unterhalb dieses Grenzwerts.

Um eine weitere Lanzette in die Gebrauchsposition zu befördern, kann die Sperre gelöst werden, was beispielsweise durch erneutes Betätigen der Betätigungseinrichtung erreicht werden kann.

Bevorzugt umfasst die Fördereinrichtung eines erfindungsgemäßen Stechsystems eine Wickeleinrichtung, die das Band aufwickelt und auf diese Weise in Förderrichtung bewegt. Bei einer derartigen Wickeleinrichtung ändert sich der zum Transport einer Lanzette in die Gebrauchsposition erforderliche Drehwinkel, da mit zunehmendem Aufwickeln des Trägerbandes wegen des zunehmenden Durchmessers der Bandwicklung der durch eine Umdrehung bewirkte Bandtransport zunimmt. Um die zum Positionieren einer Lanzette erforderliche Drehbewegung der Wickeleinrichtung zu bewirken, genügt deshalb eine umso kleinere Antriebsbewegung der Betätigungseinrichtung je mehr Lanzetten eines Trägerbandes bereits benutzt wurden. Mit einer Rutschkupplung kann hierfür in vorteilhafter Weise eine immer wieder gleiche Antriebsbewegung der Betätigungseinrichtung genutzt werden, da nach Blockieren des Weitertransports die Rutschkupplung durchrutscht und die Fördereinrichtung von der restlichen Antriebsbewegung der Betätigungseinrichtung nicht mehr beeinflusst wird.

Eine gleich bleibende Antriebsbewegung lässt sich beispielsweise dadurch bewirken, dass die Betätigungseinrichtung eine Stange umfasst, die zur Betätigung von einem Benutzer in ihrer Längsrichtung verschoben wird. Im einfachsten Fall entspricht die Länge der Antriebsbewegung dabei dem Weg, um den die Stange in Längsrichtung verschoben wird, beispielsweise indem ein aus dem Gehäuse herausragendes Ende der Stange stets um denselben Weg in das Gehäuse gedrückt wird.

Weitere Einzelheiten und Vorteile der Erfindung werden anhand von Ausführungsbeispielen unter Bezugnahme auf die beigefügten Zeichnungen erläutert. Gleiche und einander entsprechende Bauteile sind dabei mit übereinstimmenden Bezugszahlen gekennzeichnet. Die beschriebenen Merkmale können einzeln oder in Kombination zum Gegenstand von Ansprüchen gemacht werden. Es zeigen:
- Figur 1: eine schematische Darstellung eines Stechsystems;
- Figur 2: eine Detailansicht des Stechantriebs mit einem Lanzettenträgerband vor Erreichen der Gebrauchsposition;
- Figur 3: eine Schnittansicht zu Figur 2 entlang der Schnittlinie A-A;
- Figur 4: eine Ansicht gemäß Figur 2 mit einer Lanzette in der Gebrauchsposition;
- Figur 5: eine Schnittansicht zu Figur 4 entlang der Schnittlinie A-A;
- Figur 6: eine Detailansicht des Stechantriebs und eines Trägerbandes vor Erreichen der Gebrauchsposition mit einem alternativen Ausführungsbeispiel eines Sperrmechanismus;
- Figur 7: eine Schnittansicht zu Figur 6 entlang der Schnittlinie A-A;
- Figur 8: eine Ansicht gemäß Figur 6 mit der Lanzette in der Gebrauchsposition;
- Figur 9: eine Schnittansicht zu Figur 8 entlang der Schnittlinie A-A;
- Figur 10: eine schematische Darstellung eines Stechsystems mit einem Aktuator zum Lösen der Sperre;
- Figur 11: ein Ausführungsbeispiel einer Kulisse zur Steuerung des Aktuators;
- Figur 12: eine schematische Darstellung des Aktuators in einer Schnittdarstellung durch das Trägerband;
- Figur 13: eine Darstellung gemäß Figur 12 bei geöffneter Sperre;
- Figur 14: ein weiteres Ausführungsbeispiel eines Stechsystems.

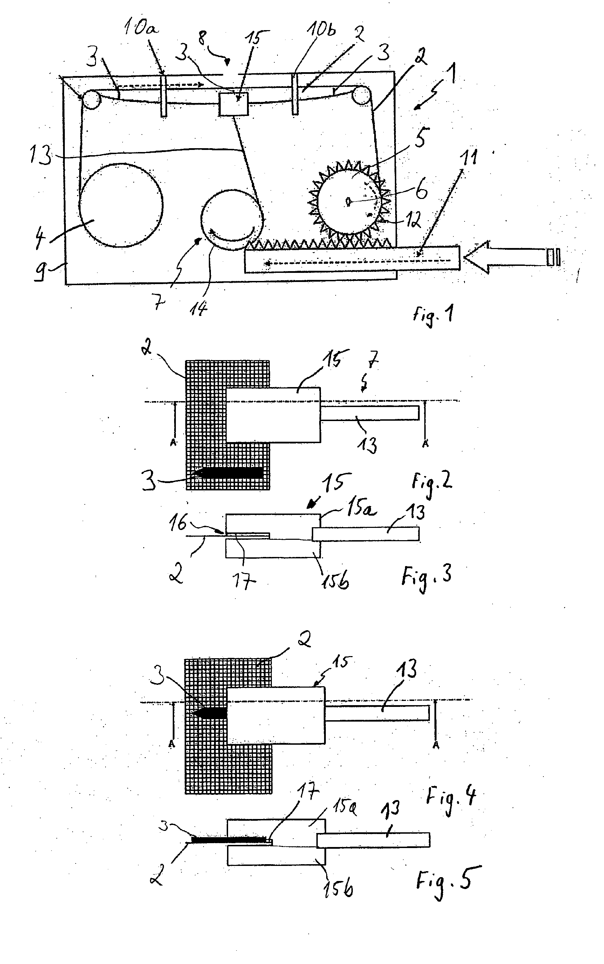

Figur 1 zeigt eine schematische Darstellung eines Stechsystems 1. Zu dem Stechsystem 1 gehört ein Trägerband 2, das mehrere Lanzetten 3 trägt, die quer zur Längsrichtung des Bandes angeordnet sind. Mit einer Fördereinrichtung 6, die bei dem dargestellten Ausführungsbeispiel eine Wickeleinrichtung zum Antreiben einer Rolle 5 ist, kann das Trägerband 2 aufgewickelt und dabei in einer Förderrichtung bewegt werden, so dass die Lanzetten 3 des Trägerbandes 2 nacheinander in eine Gebrauchsposition gelangen, in der sie mit einem Stechantrieb 7 für eine Stechbewegung beschleunigt werden können, um in ein an eine Gehäuseöffnung 8 angelegtes Körperteil einzustechen.

Das Trägerband 2 ist bei dem dargstellten Ausführungsbeispiel ähnlich wie bei einer Tonbandkassette auf eine Vorratsrolle 4 aufgewickelt, von der es abgewickelt und bei Verbrauch der Lanzetten 3 nach und nach auf die angetriebene Wickelrolle 5 aufgewickelt wird. Das Trägerband 2 kann austauschbar in einem Gehäuse 9 eines Stechgeräts angeordnet sein, beispielsweise als Teil einer Kassette, oder in einem Gerät fest eingebaut sein, das bestimmungsgemäß entsorgt wird, sobald alle Lanzetten 3 des Trägerbandes 2 aufgebraucht sind. Anstelle der Vorratsrolle 4 kann beispielsweise auch ein Stapel verwendet werden, zu dem das Trägerband 2 zickzackförmig gefaltet ist.

Beim Bandtransport läuft das Trägerband 2 mit einer Lanzette 3, bevor diese die Gebrauchsposition erreicht, an einer Umlenkeinrichtung 10a vorbei, die ein Verdrehen des Trägerbands 2 um 90° bewirkt. Nach Gebrauch einer Lanzette 3 wird der betreffende Abschnitt des Trägerbandes 2 an einer zweiten Umlenkeinrichtung 10b vorbeigeführt, mit der das Trägerband 2 wieder in seine ursprüngliche Orientierung zurückgedreht wird.

Die Fördereinrichtung 6 wird von einer Betätigungseinrichtung 11 angetrieben, die bei dem dargestellten Ausführungsbeispiel als Zahnstange ausgebildet ist, die mit einem Zahnrad 12 in Eingriff steht. Um eine frische Lanzette 3 in die Gebrauchsposition zu befördern, betätigt ein Benutzer die Betätigungseinrichtung 11, bei dem dargestellten Ausführungsbeispiel indem diese in Richtung des dargestellten Pfeils in das Gehäuse 9 hineingedrückt, also in ihrer Längsrichtung verschoben wird.

Die Betätigungseinrichtung 11 ist über eine Rutschkupplung an die Fördereinrichtung 6 gekoppelt. Bei dem dargestellten Ausführungsbeispiel wirkt die nicht dargestellte Rutschkupplung zwischen dem Zahnrad 12 und der Wickeleinrichtung 6. Sobald eine Lanzette 3 die Gebrauchsposition erreicht hat, blockiert eine lösbare Sperre einen Weitertransport des Trägerbandes 2. Die Rutschkupplung rutscht daraufhin durch, so dass kein Drehmoment mehr auf die Wickeleinrichtung 6 übertragen wird.

Die Betätigungseinrichtung 11 kann auf diese Weise bei jeder Betätigung eine gleich bleibende Antriebsbewegung durchführen, obwohl sich der zum Transport einer Lanzette 3 in die Gebrauchsposition erforderliche Drehwinkel der Wickeleinrichtung 6 mit zunehmenden Aufwickeln des Trägerbandes 2 auf die Wickelrolle 5 ändert, nämlich abnimmt.

Die Betätigungseinrichtung 11 ist auch mit dem Stechantrieb 7 gekoppelt. Der Stechantrieb 7 hat einen Energiespeicher, der die für eine Stechbewegung erforderliche Energie liefert und durch Betätigen der Betätigungseinrichtung 11 aufgeladen wird. Bei dem dargestellten Ausführungsbeispiel ist der Energiespeicher eine Feder, bevorzugt eine Kunststofffeder, beispielsweise ein Gummiband. Bei dem dargestellten Ausführungsbeispiel erfolgt die Kopplung der Betätigungseinrichtung 11 mit dem Energiespeicher über ein Rad 14, das durch eine Bewegung der Betätigungseinrichtung 11 in Rotation versetzt wird und diese Bewegung über eine Pleuelstange 13 auf einen Kopplungskopf 15 überträgt, der das Trägerband 2 hält.

Eine Sperre, die einen Weitertransport des Trägerbandes 2 blockiert, sobald eine Lanzette 3 die Gebrauchsposition erreicht hat, kann auf unterschiedliche Weise verwirklicht werden. Ein erstes Ausführungsbeispiel für eine geeignete Sperre wird im Folgenden anhand der Figuren 2 bis 5 erläutert.

Figur 2 zeigt eine Detailansicht zu Figur 1, in der ein Abschnitt des Trägerbandes 2 mit einer Lanzette 3 vor Erreichen der Gebrauchsposition sowie einen Kopplungskopf 15 des Stechantriebs 7, mit dem eine von dem Stechantrieb 7 erzeugte Kraft für eine Stechbewegung auf eine Lanzette 3 und das mit ihr verbundene Trägerband 2 übertragen wird. Figur 3 zeigt eine Schnittdarstellung zu Figur 2 entlang der Schnittlinie A-A. Wie Figur 3 zeigt, hat der Kopplungskopf 15 einen Spalt 16, durch den das Trägerband 2 hindurchgeführt wird. Der Spalt 16 wird zwischen zwei gegeneinander beweglichen Teilen 15a, 15b des Kopplungskopfs 15 gebildet. Diese beiden Teile werden durch Federkraft gegeneinander gedrückt. Fertigungstoleranzen können so vorteilhaft ausgeglichen werden.

In der in Figur 3 dargestellten Lage der beiden Kopplungsteile 15a und 15b zueinander hat der Spalt 16 eine Engstelle, durch die das Trägerband 2, nicht jedoch eine an dem Trägerband 2 befestigte Lanzette 3 hindurchpasst. Sobald eine Lanzette 3 die Gebrauchsposition erreicht, wird sie und damit auch das mit ihr verbundene Trägerband 2 durch die Engstelle des Kopplungskopfs 15 blockiert. Dies ist in den Figuren 4 und 5 gezeigt.

Die Engstelle des Spaltes 16 wird durch eine Schulter mit einer Anschlagfläche 17 des Kopplungsteils 15a gebildet. In der Gebrauchsposition schlägt die Lanzette 3 an die Anschlagfläche 17 an. Das Kopplungsteil 15a mit der Anschlagfläche 17 bildet auf diese Weise ein Sperrelement, das mit einer Lanzette 3 in der Gebrauchsposition zusammenwirkt. Die Lanzette 3 hat einen Lanzettenkörper, der auf dem Trägerband 2 eine quer zur Förderrichtung verlaufende Stufe bildet, die als Blockadeelement zum Positionieren einer Lanzette 3 in der Gebrauchsposition mit der Anschlagfläche 17 der Sperre zusammenwirkt.

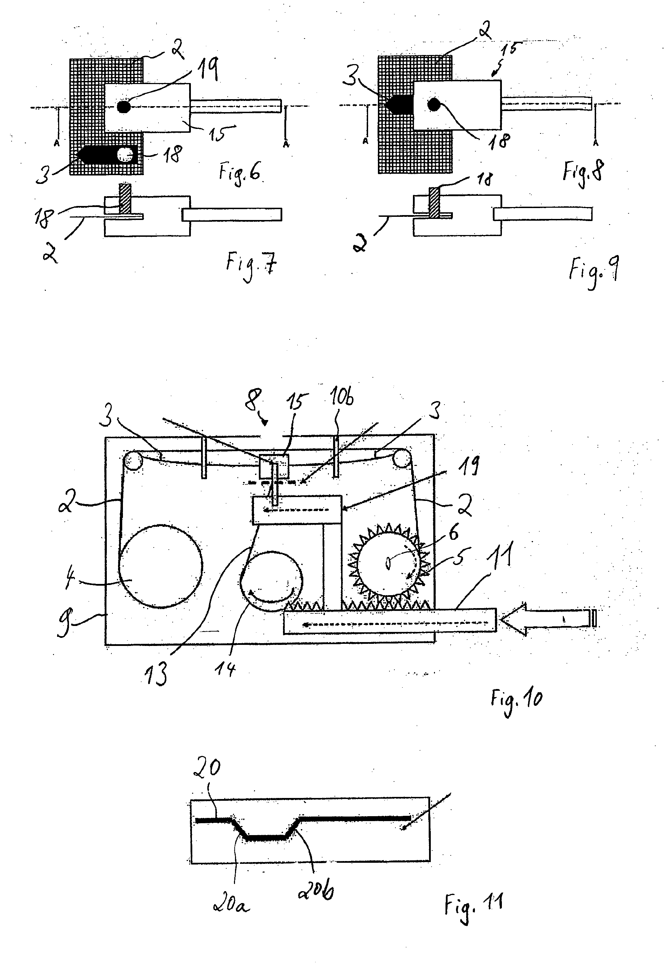

Beim Auslösen eines Stichs wird das Betätigungselement 11 in seine ursprüngliche Position zurückversetzt, beispielsweise mittels einer Rückstellfeder. Bei einer erneuten Betätigung des Betätigungselements 11 wird die Sperre gelöst, indem die beiden Kopplungselemente 15a und 15b gegeneinander bewegt werden, so dass sich der Spalt 16 vorübergehend verbreitert und das Trägeband 2 weiter transportiert werden kann. Ein Beispiel wie dies bewirkt werden kann, wird später anhand der Figuren 10 bis 13 erläutert.

Die Figuren 6 bis 9 zeigen ein weiteres Beispiel, wie eine Sperre bewirkt werden kann, die einen Weitertransport des Trägerbandes 2 blockiert, sobald eine Lanzette 3 die Gebrauchsposition erreicht hat. Bei diesem Beispiel ist in der Lanzette 3 ein Loch 18 als Blockadeelement vorgesehen, das mit einem als Zapfen ausgebildeten Sperrelement 19 zusammenwirkt, das in dem Kopplungsteil 15 angeordnet ist. Der Bolzen 19 ist senkrecht zur Ebene des Trägerbands 2 beweglich. Gelangt eine Lanzette 3 in die Gebrauchsposition, greift der Bolzen 19 in das Loch 18 der Lanzette 3 ein, so dass die Lanzette 3 und das mit ihr verbundene Trägerband 2 blockiert sind.

Um zuverlässig in das Loch 18 der Lanzette 3 einzugreifen kann der Bolzen 19 beispielsweise mit Federkraft gegen das Band 2 gedrückt sein. Eine Schrägfläche an seinem in das Loch 18 eingreifenden Ende bewirkt, dass der Bolzen 19 von einer in die Gebrauchsposition gelangenden Lanzette 3 angehoben werden kann. Sobald sich die Lanzette 3 in der Gebrauchsposition befindet, greift der Bolzen 19 dann in das Loch 18 der Lanzette 3 ein. Das Loch 18 der Lanzette 3 kann als Sackloch ausgebildet sein. Bevorzugt ist jedoch ein durchgehendes Loch.

Bei dem anhand der Figuren 6 bis 9 erläuterten Sperrmechanismus bildet der Bolzen 19 das Sperrelement der Sperre, das mit einer Lanzette 3 in der Gebrauchsposition formschlüssig zusammenwirkt. Die Umfangsfläche des Bolzens 19 bildet dabei eine Anschlagfläche, an die eine Lanzette 3 in der Gebrauchsposition anschlägt.

Indem ein Sperrelement der Sperre in den Kopplungskopf 15, der eine Bewegung des Stechantriebs auf das Trägerband 2 überträgt, integriert wird, kann eine besonders präzise Positionierung einer Lanzette 3 in der Gebrauchsposition erreicht werden, da dann nur bei wenigen Teilen Fertigungstoleranzen die Positioniergenauigkeit beeinflussen. Bei einem Sperrelement in dem Kopplungskopf 15 ist die Toleranzkette vorteilhaft kurz.

Das Loch 18 kann auch in dem Trägerband 2 neben einer Lanzette 3 angeordnet sein. Bei der Herstellung eines Trägerbandes 2 ist dann darauf zu achten, dass die Lanzetten 3 stets an einer vorgegebenen Position in Relation zu einem Loch 18 angeordnet sind. Dies kann dadurch erreicht werden, dass derartige Löcher erst nach dem Aufbringen der Lanzetten 3 in einer vorgegebenen Position in Relation zu den Lanzetten 3 in dem Trägerband 2 erzeugt werden. Möglich ist es auch, in einem Trägerband 2 zuerst Löcher zu erzeugen und die Lanzetten 3 in einer vorgegebenen Position relativ zu den Löchern anzuordnen.

Figur 10 zeigt eine weitere schematische Darstellung eines Stechsystems. Figur 10 unterscheidet sich von Figur 1 im wesentlichen durch einen Aktuator 19, der an die Betätigungseinrichtung 11 gekoppelt ist und dazu dient, die Sperre aus einer Sperrposition, in der ein Weitertransport des Förderbandes 2 blockiert wird, in eine Durchlassposition, in der Förderband 2 weitertransportiert werden kann, und wieder zurück in die Sperrposition zu überführen. Sperrpositionen sind beispielsweise in den Figuren 5 und 9 für unterschiedliche Ausführungsbeispiele der Sperre dargestellt.

Der Aktuator 19 ist bei dem dargestellten Ausführungsbeispiel über eine Kulissensteuerung an die Betätigungseinrichtung 11 gekoppelt. Ein Beispiel der Kulisse, die bei Betätigung der Betätigungseinrichtung 11 abgefahren wird, ist in Figur 11 gezeigt. Die Kulisse 20 bildet eine Steuerkurve, die von einem Steuerkurvenreiter abgefahren wird und dabei ein kurzes Anheben des als Bolzen 19 ausgebildeten Sperrelements bzw. eine Relativbewegung der beiden Kopplungsteile 15a, 15b zueinander bewirkt. Der Aktuator 19 wird gleichzeitig mit der Fördereinrichtung 6 von der Betätigungseinrichtung 11 betätigt, so dass während des kurzzeitigen Öffnens der Sperre eine benutzte Lanzette 3 aus der Gebrauchsposition weiterbewegt wird. Bevor eine frische Lanzette 3 die Gebrauchsposition erreicht, ist die Sperre in die Sperrposition zurückgekehrt, so dass die nächste Lanzette 3, sobald diese die Gebrauchsposition erreicht hat, zusammen mit dem mit ihr verbundenen Förderband 2 blockiert wird.

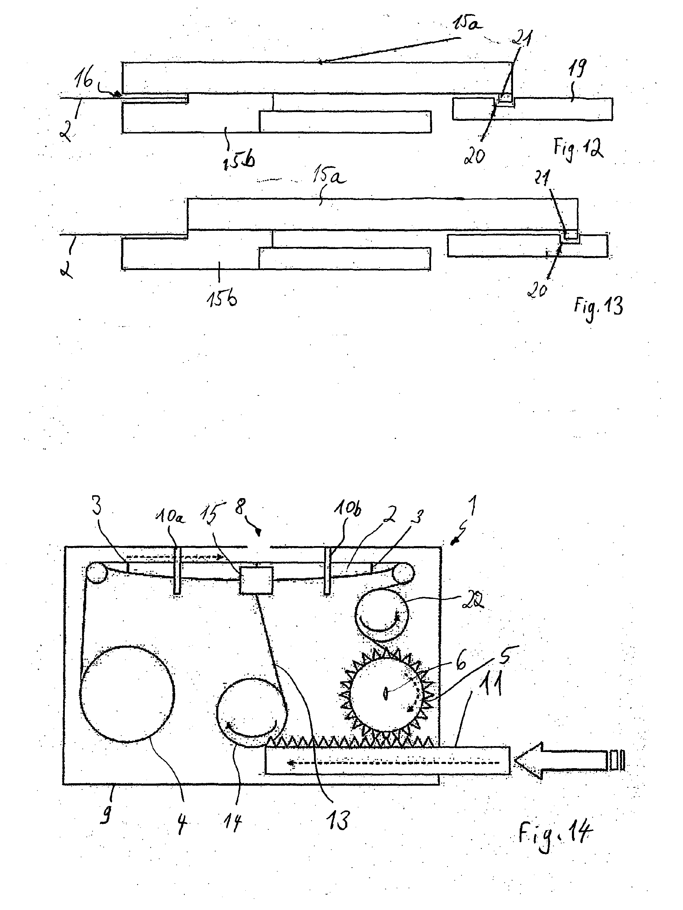

Figur 12 zeigt eine Schnittansicht des aus den beiden Teilen 15a, 15b gebildeten Kopplungskopfs 15 sowie des Aktuators 19 mit der als Nut ausgebildeten Kulisse 20. In die Kulisse 20 greift ein Stift 21 als Steuerkurvenreiter ein, der mit dem Kopplungsteil 15a verbunden ist. In Figur 12 ist die Sperre in der Sperrposition dargestellt. Beim Abfahren des Steuerkurvenabschnitts 20a der Kulisse 20 durch den Stift 21 vollzieht das Kopplungsteil 15a die durch die Kulisse 20 vorgegebene Bewegung nach und wird deshalb entgegen der Stichrichtung zurückgezogen, so dass die in Figur 13 gezeigte Position erreicht wird, in der das Trägerband 2 und eine von ihm getragene Lanzette 3 nicht mehr in dem Spalt 16 eingeklemmt sind. Durch den darauf folgenden Steuerkurvenabschnitt 20b wird die Sperre wieder in ursprüngliche Position, die in Figur 12 gezeigt ist, zurück versetzt.

Neben einer Kopplung des Aktuators an die Betätigungseinrichtung über eine zweiseitig geführte Kurvensteuerung, bei der ein Steuerkurvenreiter in eine Nut eingreift, besteht auch die Möglichkeit einer einseitig geführten Kurvensteuerung zur Kopplung des Aktuators an die Betätigungseinrichtung. Bei einer einseitig geführten Kurvensteuerung wird der Steuerkurvenreiter an eine Steuerkurve angedrückt, die beispielsweise eine Oberfläche mit Erhöhungen und Vertiefungen ist, über die der Steuerkurvenreiter beim Abfahren der Steuerkurve gleitet. Im Einfachsten Fall kann die Steuerkurve beispielsweise eine Rampe sein.

Figur 14 zeigt ein weiteres Ausführungsbeispiel eines Stechsystems 1. Dieses Ausführungsbeispiel unterscheidet sich von dem vorstehend erläuterten Ausführungsbeispiel dadurch, dass das Trägerband 2 mit einem Zählrad 22 gekoppelt ist, das sich bei einer durch die Fördereinrichtung 6 bewirkten Bewegung des Trägerbandes 2 zur Messung einer von dem Trägerband 2 zurückgelegten Wegstrecke dreht und blockiert, sobald die gemessene Wegstrecke die zum Positionieren einer Lanzette 3 in der Gebrauchsposition erforderliche Länge erreicht hat. Das Zählrad 22 bildet bei diesem Ausführungsbeispiel also die Sperre. Bevorzugt ist der Umfang des Zählrads 22 so bemessen, dass er genau den Abstand zwischen benachbarten Lanzetten 3 auf dem Trägerband 2 entspricht und das Zählrad 22 deshalb jeweils nach einer vollen Umdrehung sperrt. Möglich ist es selbstverständlich auch, dass das Zählrad 22 bei einer beliebigen anderen vorgegebenen Drehwinkelstellung oder einer vorgegebenen Anzahl von Umdrehungen sperrt. Durch Reibschluss oder eine mit dem Zählrad 22 zusammenwirkende Perforation des Trägerbandes 2 kann Schlupf des Trägerbandes 2 vermieden werden.

### Bezugszahlenliste

- 1: Stechsystem
- 2: Trägerband
- 3: Lanzette
- 4: Vorratsrolle
- 5: Wickelrolle
- 6: Fördereinrichtung
- 7: Stechantrieb
- 8: Gehäuseöffnung
- 9: Gehäuse
- 10a: Umlenkeinrichtung
- 10b: Umlenkeinrichtung
- 11: Betätigungseinrichtung
- 12: Zahnrad
- 13: Pleuelstange
- 14: Rad
- 15: Kopplungskopf
- 15a: Teil des Kopplungskopfs
- 15b: Teil des Kopplungskopfs
- 16: Spalt
- 17: Anschlagfläche
- 18: Loch
- 19: Aktuator
- 20: Kulisse
- 20a: Steuerkurvenabschnitt
- 20b: Steuerkurvenabschnitt
- 21: Stift
- 22: Zählrad

## Patentansprüche

1. Stechsystem mit einem Trägerband (2), das mehrere Lanzetten (3) trägt,
einem Gerätegehäuse (9), das eine Fördereinrichtung (6), um die Lanzetten (3) durch Bewegen des Trägerbandes (2) in einer Förderrichtung nacheinander in eine Gebrauchsposition zu bringen, und einen Stechantrieb (7), um eine in der Gebrauchsposition positionierte Lanzette (3) für eine Stechbewegung zu beschleunigen, enthält und eine Gehäuseöffnung (8) zum Anlegen eines Körperteils, in das mit einer Stechbewegung einer Lanzette (3) eingestochen werden soll, aufweist, und
eine Betätigungseinrichtung (11) zum Antreiben der Fördereinrichtung (6),
**gekennzeichnet durch**
eine lösbare Sperre (17, 18, 22), die einen Weitertransport des Trägerbandes (2) blockiert, sobald eine Lanzette (3) die Gebrauchsposition erreicht hat.

2. Stechsystem nach Anspruch 1, **dadurch gekennzeichnet, dass** die Sperre (17, 18, 21) durch erneutes Betätigen der Betätigungseinrichtung (11) lösbar ist.

3. Stechsystem nach einem der vorstehenden Ansprüche, **gekennzeichnet durch** eine Rutschkupplung, über welche die Betätigungseinrichtung (11) an die Fördereinrichtung (6) gekoppelt ist,

4. Stechsystem nach einem der vorstehenden Ansprüche, **dadurch gekennzeichnet, dass** der Antrieb einen Energiespeicher umfasst, der die für eine Stechbewegung erforderliche Energie liefert und mit der Betätigungseinrichtung (11) gekoppelt ist, so dass durch Betätigen der Betätigungseinrichtung (11) ein Aufladen des Energiespeichers bewirkt wird.

5. Stechsystem nach einem der vorstehenden Ansprüche, **dadurch gekennzeichnet, dass** die Sperre ein Sperrelement (17, 18) umfasst, das mit einer Lanzette (3) in der Gebrauchsposition zusammenwirkt.

6. Stechsystem nach Anspruch 4, **dadurch gekennzeichnet, dass** das Sperrelement (17, 18) den mit einer Lanzette (3) in der Gebrauchsposition formschlüssig zusammenwirkt.

7. Stechsystem nach Anspruch 4 oder 5, **dadurch gekennzeichnet, dass** das Sperrelement (17, 18) eine Anschlagfläche aufweist, an die eine Lanzette (3) in der Gebrauchsposition anschlägt.

8. Stechsystem nach einem der vorstehenden Ansprüche, **dadurch gekennzeichnet, dass** die Sperre (17, 18, 22) mittels eines an die Betätigungseinrichtung (11) gekoppelten Aktuators (19) aus einer Sperrposition, in der ein Weitertransport des Förderbandes (2) blockiert wird, in eine Durchlassposition, in der das Förderband (2) weitertransportiert werden kann, überführbar ist.

9. Stechsystem nach Anspruch 7, **dadurch gekennzeichnet, dass** der Aktuator (19) über eine Kurvensteuerung (20) an die Betätigungseinrichtung (11) gekoppelt ist.

10. Stechsystem nach einem der vorstehenden Ansprüche, **dadurch gekennzeichnet, dass** die Sperre ein mit dem Trägerband (2) gekoppeltes Zählrad (22) umfasst, das sich bei einer durch die Fördereinrichtung (6) bewirkten Bewegung des Trägerbandes (2) zur Messung einer von dem Trägerband (2) zurückgelegten Wegstrecke dreht und blockiert, sobald die Wegstrecke die zum Positionieren einer Lanzette (3) in der Gebrauchsposition erforderliche Länge erreicht hat.

11. Stechsystem nach einem der vorstehenden Ansprüche, **dadurch gekennzeichnet, dass** die Betätigungseinrichtung (11) eine Stange umfasst, die zur Betätigung der Fördereinrichtung (6) von einem Benutzer in ihrer Längsrichtung verschoben wird.

12. Trägerband für ein Stechgerät, wobei das Trägerband (2) mehrere Lanzetten (3) trägt, **gekennzeichnet durch** Blockadeelemente (18), die zum Positionieren einer Lanzette (3) in einer Gebrauchsposition mit einer lösbaren Sperre (17, 18, 22) eines Stechgeräts zusammenwirken.
